Europäisches Patentamt

European Patent Office

Office européen des brevets

(19)

(11) Numéro de publication: **0 220 103 B1**

(12) **FASCICULE DE BREVET EUROPEEN**

(45) Date de publication du fascicule du brevet: **28.11.90**

(51) Int. Cl.⁵: **A 23 L 1/236, A 61 K 9/14**

(21) Numéro de dépôt: **86402174.6**

(22) Date de dépôt: **02.10.86**

(54) **Maltitol poudre directement compressible et son procédé de préparation.**

(30) Priorité: **02.10.85 FR 8514611**

(43) Date de publication de la demande:
**29.04.87 Bulletin 87/18**

(45) Mention de la délivrance du brevet:
**28.11.90 Bulletin 90/48**

(84) Etats contractants désignés:
**BE CH DE ES GB IT LI NL SE**

(56) Documents cités:
**EP-A-0 072 080**
**FR-A-2 236 005**
**FR-A-2 275 555**
**FR-A-2 451 357**

(73) Titulaire: **Roquette Frères
F-62136 Lestrem (FR)**

(72) Inventeur: **Serpelloni, Michel
210 Boulevard Voltaire
F-62100 Bethune (FR)**

(74) Mandataire: **Koch, Gustave et al
Cabinet PLASSERAUD 84, rue d'Amsterdam
F-75009 Paris (FR)**

Courier Press, Leamington Spa, England.

**Description**

L'invention a pour objet, à titre de produit industriel nouveau, du maltitol poudre directement compressible.

Elle a également pour objet un procédé de préparation de ce maltitol poudre.

Le pouvoir sucrant du maltitol, qui est élevé par rapport à celui des sucres-alcool classiques tels que le sorbitol, le mannitol et autres, ouvrirait des applications nombreuses au maltitol dans le domaine des industries pharmaceutiques et alimentaires s'il était possible de lui conférer des caractéristiques de compression directe acceptables.

Il a déjà été question dans la littérature scientifique et commerciale de maltitol poudre directement compressible, avec ou sans liant de granulation.

Or, en l'absence de liant, la compressibilité du produit n'est pas très satisfaisante.

Et la présence d'un liant n'est pas très prisée par les fabricants notamment de produits pharmaceutiques.

La Société Demanderesse a donc cherché à mettre au point un maltitol poudre directement compressible ne comportant pas de liant et présentant des propriétés de compressibilité améliorées par rapport à celles des produits déjà existants.

Et elle a eu le mérite de préparer effectivement un tel maltitol poudre qui est caractérisé par une richesse en maltitol supérieure à 85% en poids sur matières sèches et par une compressibilité, déterminée dans un test A, supérieure à 80 N.

De préférence,

la richesse en maltitol du susdit produit industriel nouveau est supérieure à 92% en poids et plus préférentiellement encore à 95% en poids et

sa compressibilité, déterminée dans le test A, est supérieure à 100 N.

Le test A consiste à mesurer la force, exprimée en Newton, qui est représentative de la compressibilité du maltitol étudié et qui est nécessaire pour provoquer l'écrasement d'un comprimé préparé à partir dudit maltitol, c'est-à-dire pour provoquer l'apparition de lignes de rupture au sein de la masse constitutive de celui-ci, cette force traduisant donc la résistance à l'écrasement du comprimé qui est cylindrique à faces planes d'un diamètre de 13 mm, d'une épaisseur de 5 mm et d'un poids de 0,896 g, c'est-à-dire d'une masse volumique apparente de 1,35 g/ml, ladite force étant exercée contre la surface périphérique du comprimé en direction de l'axe de révolution de celui-ci au moyen d'une butée mobile s'appliquant contre ladite surface le long d'une génératrice, ledit comprimé étant par ailleurs immobilisé contre une butée fixe appliquée également contre la surface périphérique du comprimé le long d'une génératrice diamétralement opposée à celle contre laquelle s'applique la butée mobile.

Conformément au procédé mis au point par la Société Demanderesse pour préparer le maltitol poudre constituant le susdit produit industriel nouveau, on soumet une matière première essentiellement constituée de maltitol poudre à un traitement d'extrusion à l'intérieur d'une installation de cuisson-extrusion comprenant une zone de chauffage et une filière d'extrusion, le débit d'alimentation de l'installation en matière première ainsi que les paramètres du traitement d'extrusion, à savoir la température régnant à l'intérieur de la zone de chauffage, le diamètre de la filière d'extrusion et la vitesse d'entrainement de la matière première à l'intérieur de la zone de chauffage étant sélectionnés de façon telle qu'à la sortie de la filière et avant sa sortie de cette dernière, ledit maltitol soit partiellement fondu, ledit maltitol sortant de la filière étant ensuite soumis à un broyage.

Le maltitol poudre conforme à l'invention est également défini comme étant un maltitol poudre susceptible d'être obtenu par le procédé conforme à l'invention.

De préférence, la susdite installation est du type bi-vis comportant une filière d'extrusion, les paramètres du traitement d'extrusion étant sélectionnés de façon telle que la matière première se trouve à une température de 100 à 160°C et, de préférence, de 110 à 150°C à l'intérieur de la filière et avant la sortie du maltitol de cette dernière.

Dans ces conditions, la proportion de maltitol fondu est de 30 à 90%, et plus généralement de 50 à 80%.

Le temps mis par le maltitol pour traverser l'installation d'extrusion est avantageusement de 0,5 à 10 minutes, de préférence de 1 à 4 minutes.

L'invention vise encore d'autres dispositions qui s'utilisent de préférence en même temps et dont il sera plus explicitement question ci-après et elle pourra, de toute façon, être bien comprise à l'aide du complément de description qui suit ainsi que du dessin annexé et de l'exemple, lesdits complément de description et exemple étant relatifs à des modes de réalisation avantageux.

La figure unique du dessin représente, en coupe schématique, une installation d'extrusion du type de celles pouvant être utilisées dans le cadre du procédé selon l'invention.

Se proposant, par conséquent, de fabriquer du maltitol poudre selon l'invention, directement compressible sans utilisation de liant, on s'y prend comme suit ou de façon équivalente.

La matière première qui est soumise au traitement d'extrusion selon le procédé conforme à l'invention, est constituée de maltitol poudre essentiellement sous forme de maltitol cristallisé.

Dans la pratique, on utilise des cristaux natifs de maltitol, c'est-à-dire des cristaux obtenus par cristallisation dans l'eau ou par solidification de maltitol fondu; toujours dans la pratique, on ajoute à ces cristaux natifs, par recyclage, la partie du maltitol, obtenue à l'issue du procédé, qui n'est pas utilisable commercialement, par exemple en raison d'une granulométrie mal adoptée aux besoins du marché.

La température de ces cristaux est généralement comprise, à l'entrée de l'installation d'extrusion mise en oeuvre dans le cadre du procédé selon l'invention, entre 15 et 80°C.

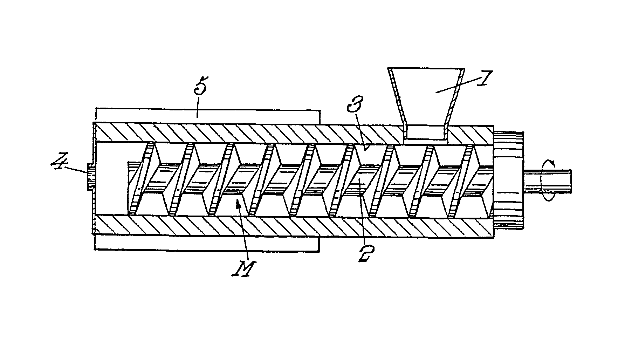

L'installation d'extrusion est constituée avantageusement par une extrudeuse du type bi-vis comprenant, comme montré à la figure 1:

un système d'alimentation, notamment une trémie doseuse et mélangeuse 1,

une zone de malaxage M comprenant un système à deux vis sans fin 2 disposées à l'intérieur d'un carter 3, notamment en acier nitruré, et entraînées en rotation par un mécanisme non montré,

une sortie comprenant une ou plusieurs filières 4 de différentes formes,

des moyens de chauffage 5 permettant de contrôler la température de la zone de malaxage, ces moyens de chauffage 5 étant constitués par exemple par des résistances électriques, par un système de chauffage par induction ou à la vapeur et par des moyens de refroidissement non montrés disposés à l'extérieur du carter ou à l'intérieur et se présentant par exemple sous la forme de serpentins logés dans le carter, d'un circuit de fluide de refroidissement logé à l'intérieur de la vis, et autres.

La matière première entrant par le système d'alimentation dans la zone de malaxage est soumise, grâce à la compression réalisée dans les spires de la vis, à un cisaillement et à un frottement mécanique intenses simultanément à l'échauffement induit par les moyens de chauffage.

L'extrusion constitue, par conséquent, un traitement thermo-mécanique.

Pour fixer les idées, on signale qu'on a obtenu de bons résultats avec une extrudeuse du type bi-vis mise sur le marché sous la dénomination "BC 82" par la Société CREUSOT-LOIRE. Les deux vis se copénètrent et tournent dans le même sens. La zone de malaxage est chauffée par induction et la température peut donc y être facilement régulée.

L'avantage essentiel de ce mode de chauffage est sa souplesse d'utilisation et son contrôle aisé au moyen d'une boucle de régulation simple (thermocouple/dispositif de commande de l'alimentation électrique des moyens de chauffage par induction). Il se pourrait que l'existence d'un champ électromagnétique intense exerce une influence sur les propriétés du produit.

Dans le cas de l'installation utilisée dans le cadre de l'exemple décrit plus loin, la filière utilisée était de forme cylindrique et avait un diamètre de 5 mm.

La température de la zone de chauffage est obtenue en imposant au système de chauffage une valeur prédéterminée. Dans le cas de l'installation d'extrusion dont il vient d'être question, cette valeur est comprise entre 210 et 280°C, de préférence entre 240 à 280°C et, plus préférentiellement encore, voisine de 260°C.

Les caractéristiques mécaniques des vis et leur vitesse de rotation sont choisies de façon telle que la durée du séjour de la matière première à l'intérieur de la zone de chauffage soit de 1,5 à 2,5 minutes.

Grâce au choix de l'ensemble de ces paramètres, la température de la matière première ayant subi le traitement, est de 110 à 150°C à l'intérieur de la filière et avant sa sortie de cette dernière.

Le maltitol obtenu à la sortie de l'installation d'extrusion est successivement soumis:

à un refroidissement,

à un broyage,

à un tamisage et

à un recyclage des fines (particules de taille trop faible pour être retenues par le plus petit tamis de l'installation) au niveau de l'alimentation de l'extrudeuse.

Avant de tester les performances en compression du maltitol ainsi obtenu, on lui fait comprendre un agent lubrifiant, en l'occurrence du stéarate de magnésium à raison généralement de 0,5 à 2%.

## EXEMPLES

Dans les exemples qui suivent, on teste la compressibilité de deux qualités de maltitol poudre conforme à l'invention et de quatre variétés de maltitol poudre de l'art antérieur.

Pour préparer les deux qualités de maltitol conforme à l'invention, on alimente une extrudeuse du type "BC 82" dont il a été question plus haut en maltitol poudre.

La vitesse des vis est réglée de manière telle que le débit de l'installation soit de 250 kg/heure et que le temps de passage de la matière première dans l'installation soit de 2 minutes.

La température de consigne du système de chauffage est programmée à 260°C, ce qui permet d'obtenir à la sortie de l'extrudeuse du maltitol à une température variant de 145 à 147°C.

Ce maltitol se présente sous la forme de bâtonnets qui sont broyés à l'aide d'un broyeur du type à marteaux.

Par tamisage, on sépare les fines qui sont recyclées au niveau de l'alimentation de l'extrudeuse.

La fraction retenue est de granulométrie supérieure à 50 µm, plus généralement à 100 µm.

La première des deux qualités de maltitol poudre conforme à l'invention, désignée par 1a, est obtenue en utilisant comme matière première du maltitol, cristallisé dans l'eau, d'une richesse en maltitol de 98% en poids sur matières sèches.

La seconde qualité, désignée par 1b, est obtenue en utilisant du maltitol, cristallisé dans l'eau, d'une richesse en maltitol de 95% en poids sur matières sèches.

3

Les quatre qualités de maltitol poudre selon l'art antérieur, respectivement désignées par 2a, 2b, 2c et 2d, sont constituées

en ce qui concerne la qualité 2a par du maltitol cristallisé dans l'eau présentant une pureté chimique de 98% en poids,

en ce qui concerne la qualité 2b par du maltitol cristallisé dans l'eau présentant une pureté chimique de 95% en poids,

en ce qui concerne la qualité 2c par du maltitol massé présentant une pureté chimique de 92% en poids,

en ce qui concerne la qualité 2d par du maltitol massé présentant une pureté chimique de 85% en poids.

Pour les six variétés de maltitol poudre testées, on détermine la répartition granulométrique moyenne correspondant à 50% de la distribution en masse.

On mesure également leur indice d'écoulement en ayant recours à la méthode de CARR telle que décrite par CARR R.L. dans Chem. Eng. 72, No. 1, 163, 168, (1965) et Chem. Eng. 72, No. 2, 69—73 (1965); on utilise, pour ce faire, un appareil connu sous la marque "HOSOKAWA POWDER TESTER" et fabriqué par MICROMERITICS, Osaka (Japon).

On détermine de plus la friabilité de ces six qualités. Cette propriété est caractérisée comme étant représentée par le pourcentage de particules n'ayant pas résisté à un concassage dans un appareil appelé friabilimetre. En l'occurrence, on a utilisé celui de marque "ERWEKA TA". Cet appareil contient 5 billes d'acier identiques de 1,7 cm de diamètre et de 18,87 g chacune. On y introduit 15 g d'une fraction granulométrique de 400 à 500 microns de la poudre testée et on met l'appareil en rotation à 25 tours/minute pendant 15 minutes. On détermine par pesée, à la fin du concassage, la proportion, exprimée en %, que représente le résidu retenu par un tamis de largeur de maille de 351 microns; la valeur de la friabilité correspond au complément à 100 de cette dernière valeur. Plus le chiffre ainsi obtenu est grand, plus la friabilité est grande.

Enfin, on détermine par le test A défini plus haut la compressibilité de ces six qualités.

Pour la fabrication des comprimés utilisés dans le test A, on peut avoir recours à une presse alternative du type A.M. fabriquée par la Société FROGERAIS (France). Les réglages de ladite presse sont faits de telle sorte que les comprimés obtenus présentent une masse volumique apparente de 1,35 g/ml.

On ajoute à la matière première constituée par les six qualités testées, respectivement 1% en poids de lubrifiant constitué par du stéarate de magnésium.

Dans le cas des qualités 2a à 2d, on est obligé en outre d'ajouter pour la fabrication des comprimés, un agent "anticollage" ou "anti-sticking" propre à empêcher l'adhésion des comprimés sur les outils de formage; cet agent anticollage peut être du talc; il est généralement ajouté en une proportion de 3 à 7, plus généralement de 4 à 6% en poids.

Les qualités 1a et 1b ne nécessitent pas la présence d'un agent anticollage; elles sont exemptes d'agent anticollage ou au moins n'en contiennent pas une quantité sensible.

Les susdits comprimés se présentent sous la forme de cylindres de révolution ayant les dimensions suivantes:

diamètre: 13 mm

hauteur: 5 mm

poids: 0,896 g.

Pour déterminer, dans le cadre du test A, la résistance à l'écrasement des comprimés ainsi préparés, on peut avoir recours par exemple à un duromètre SCHLEUNIGER — 2E commercialisé en France par les Etablissements FROGERAIS.

Les résultats de ces mesures de compressibilité ainsi que la répartition granulométrique, la granulométrie moyenne, la teneur en agent anticollage constitué par du talc, les indices d'écoulement et la friabilité des six qualités testées, sont réunis dans le tableau ci-après.

EP 0 220 103 B1

## TABLEAU

| | Maltitol poudre conforme à l'invention | | Maltitol poudre de l'art antérieur | | | |
|---|---|---|---|---|---|---|
| Exemple (variété de maltitol) | 1a | 1b | 2a | 2b | 2c | 2d |
| Richesse en maltitol | 98 % | 95 % | 98 % | 95 % | 92 % | 85 % |
| Talc | 0 | 0 | 4 % | 6 % | 4 % | 6 % |
| Ecoulement (CARR) | 79 | 82 | > 50 | > 50 | 79 | 76 |
| Friabilité (en %) | 80,7 | 58 | - | - | 48 | 48 |
| Granulométrie | Reconstituée | Reconstituée | Reconstituée | Reconstituée | Reconstituée | Reconstituée |
| % fraction > 1000 µm | 0 | 0 | 0 | 0 | 0 | 0 |
| % fraction > 800 µm | 30 | 30 | 30 | 30 | 30 | 30 |
| % fraction > 500 µm | 30 | 30 | 30 | 30 | 30 | 30 |
| % fraction > 315 µm | 30 | 30 | 30 | 30 | 30 | 30 |
| % fraction > 200 µm | 10 | 10 | 10 | 10 | 10 | 10 |
| Granulométrie moyenne (en µm) | 580 | 580 | 580 | 580 | 580 | 580 |
| Compressibilité (résistance à la rupture) en N | 140 | 105 | 41 | mesure impossible | 75 | 52 |

Il résulte de ce tableau que le maltitol poudre conforme à l'invention

1. présente une compressibilité ou résistance à l'écrasement nettement accrue,

2. ne nécessite nullement, et c'est là un avantage déterminant, l'addition d'un agent anticollage ou "anti-sticking" tel que le talc, la poudre obtenue après extrusion n'entraînant aucun effet de collage lors de la fabrication des comprimés, ce qui est loin d'être le cas des maltitols selon l'art antérieur (on rappelle en effet que le talc a tendance à absorber les principes actifs pharmaceutiques dont la disponibilité se trouve donc diminuée).

## Revendications

1. Maltitol poudre directement compressible, caractérisé par le fait qu'il présente une richesse en maltitol supérieure à 85% en poids et par une compressibilité, déterminée dans un test A, supérieure à 80 N.

2. Maltitol poudre directement compressible selon la revendication 1, caractérisé par le fait qu'il présente

une richesse en maltitol supérieure à 92% en poids et plus préférentiellement à 95% en poids et une compressibilité, déterminée dans le test A, supérieure à 100 N.

3. Maltitol poudre directement compressible selon la revendication 1, caractérisé par le fait qu'il ne comprend aucun agent anticollant en proportions sensibles.

4. Procédé de fabrication du maltitol poudre directement compressible selon l'une des revendications 1 à 3, caractérisé par le fait qu'on soumet une matière première essentiellement constituée de maltitol poudre à un traitement d'extrusion à l'intérieur d'une installation de cuisson-extrusion comprenant une zone de chauffage et une filière d'extrusion, le débit d'alimentation de l'installation en matière première ainsi que les paramètres du traitement d'extrusion, à savoir la température régnant à l'intérieur de la zone de chauffage, le diamètre de la filière d'extrusion et la vitesse d'entraînement de la matière première à l'intérieur de la zone de chauffage étant sélectionnés de façon telle qu'à la sortie de la filière et avant sa sortie de cette dernière, ledit maltitol soit partiellement fondu, ledit maltitol sortant de la filière étant ensuite soumis à un broyage.

5. Procédé selon la revendication 4, caractérisé par le fait que l'installation mise en oeuvre est du type bi-vis comportant une filière d'extrusion, les paramètres du traitement d'extrusion étant sélectionnés de façon telle que la matière première se trouve à une température de 100 à 160°C et, de préférence, de 110 à 150°C à l'intérieur de la filière et avant la sortie du maltitol de cette dernière.

6. Procédé selon l'une des revendications 4 et 5, caractérisé par le fait que le temps mis par le maltitol pour traverser l'installation d'extrusion est avantageusement de 0,5 à 10 minutes, de préférence de 1 à 4 minutes et, plus préférentiellement encore, de 1,5 à 2,5 minutes.

7. Maltitol poudre directement compressible susceptible d'être obtenu par le procédé selon les revendications 4 à 6.

## Patentansprüche

1. Direkt pressbares Maltitpulver, dadurch gekennzeichnet, daß es einen Maltitgehalt von höher als 85 Gew.-% und eine Komprimierbarkeit, bestimmt in einem Test A, von höher als 80 N besitzt.

2. Direkt pressbares Maltitpulver gemäß Anspruch 1, dadurch gekennzeichnet, daß es besitzt

einen Maltitgehalt von höher als 92 Gew.-% und vorzugsweise höher als 95 Gew.-% und

eine Komprimierbarkeit, bestimmt in dem Test A, von höher als 100 N.

3. Direkt pressbares Maltitpulver gemäß Anspruch 1, dadurch gekennzeichnet, daß es kein Antiklebemittel in wesentlichen Anteilen enthält.

4. Verfahren zur Herstellung von direkt pressbarem Maltitpulver gemäß einem der Ansprüche 1 bis 3, dadurch gekennzeichnet, daß man ein im wesentlichen aus Maltitpulver bestehendes Ausgangsmaterial einer Extrusionsbehandlung im Inneren einer Koch-Extrusions-Vorrichtung unterzieht, die eine Heizzone und eine Extrusionsdüse aufweist, wobei die Geschwindigkeit der Vorrichtung hinsichtlich der Zufuhr des Ausgangsmaterials sowie die Parameter der Extrusionsbehandlung, d.h. die im Inneren der Heizzone vorherrschende Temperatur, der Durchmesser der Extrusionsdüse und die Durchlaufgeschwindigkeit des Ausgangsmaterials im Inneren der Heizzone derart gewählt werden, daß am Ausgang der Düse und vor dem Ausgang dieser letzteren das Maltit teilweise geschmolzen ist, wobei das die Düse verlassende Maltit hierauf einer Zerkleinerung unterzogen wird.

5. Verfahren gemäß Anspruch 4, dadurch gekennzeichnet, daß die eingesetzte Vorrichtung vom eine Extrusionsdüse aufweisenden Zweischrauben-Typ ist, wobei die Parameter der Extrusionsbehandlung derart gewählt werden, daß das Ausgangsmaterial bei einer Temperatur von 100 bis 160°C und vorzugsweise 110 bis 150°C im Inneren der Düse und vor dem Austritt des Maltits aus dieser letztgenannten vorliegt.

6. Verfahren gemäß einem der Ansprüche 4 und 5, dadurch gekennzeichnet, daß die Zeit, die aufgewendet wird, damit das Maltit die Extrusionsvorrichtung durchläuft, vorteilhaft 0,5 bis 10 Minuten, vorzugsweise 1 bis 4 Minuten und insbesondere 1,5 bis 2,5 Minuten, beträgt.

7. Direkt pressbares Maltitpulver, erhältlich nach dem Verfahren gemäß den Ansprüchen 4 bis 6.

# EP 0 220 103 B1

**Claims**

1. Directly compressible powdered maltitol characterized by the fact that it has a richness in maltitol higher than 85% by weight and a compressibility, determined in a test A, higher than 80 N.

2. Directly compressible powdered maltitol according to claim 1, characterized by the fact that it has a richness in maltitol higher than 92% by weight and more preferably higher than 95% by weight and a compressibility, determined in test A, higher than 100 N.

3. Directly compressible powdered maltitol according to claim 1, characterized by the fact that it is free of any antisticking agent in appreciable proportions.

4. Process of manufacturing compressible powdered maltitol according to one of claims 1 to 3, characterized by the fact that it comprises subjecting a raw material essentially constituted by powdered maltitol to an extrusion treatment inside a cooking-extrusion installation comprising a heating zone and an extrusion die, the supply flow rate of the installation in raw material as well as the parameters of the extrusion treatment, namely the temperature existing inside the heating zone, the diameter of the extrusion die and the moving speed of the raw material inside the heating zone being selected so that at the exit of the die and before its exit from the latter, the said maltitol is partly molten, said maltitol going out from the die being then submitted to a crushing.

5. Process according to claim 4, characterized by the fact that the cooking-extrusion installation is of the dual-screw type comprising an extrusion die, the parameters of the extrusion treatment being selected so that the raw material is at a temperature of 100 to 160°C inside the die and before the exit of the maltitol from the latter.

6. Process according to one of claims 4 and 5, characterized by the fact that the time taken by the maltitol to move through the extrusion installation is from 0.5 to 10 minutes, preferably from 1 to 4 minutes and, more still preferably, from 1.5 to 2.5 minutes.

7. Directly compressible powdered maltitol obtainable by the process according to claims 4 to 6.

EP 0 220 103 B1